# EUROPÄISCHE PATENTSCHRIFT

(11) **EP 1 267 174 B1**
(45) Veröffentlichungstag und Bekanntmachung des Hinweises auf die Patenterteilung: **17.09.2014**
(21) Anmeldenummer: 02011095.3
(22) Anmeldetag: 17.05.2002
(51) Int. Cl.: G01R 33/34, G01R 33/341

(54) **Hochfrequenz-Spulenanordnung für ein Kernspintomographie-Gerät und Kernspintomographie-Gerät**
High-frequency coil assembly for a nuclear magnetic resonance imaging device, and NMR imaging device
Système de bobines à haute fréquence pour un appareil pour tomographie à spin nucléaire, et installation d'imagerie par RMN

(30) Priorität: 30.05.2001 DE 10126338
(43) Veröffentlichungstag der Anmeldung: 18.12.2002
(73) Patentinhaber: Siemens Aktiengesellschaft, 80333 München (DE)
(72) Erfinder: Reykowski, Arne, Dr., 91054 Erlangen (DE)

(56) Entgegenhaltungen:
- EP-A- 1 090 594
- US-A- 5 583 438
- US-A- 5 917 324
- PATENT ABSTRACTS OF JAPAN Bd. 1997, Nr. 09, 30. September 1997 (1997-09-30) -& JP 09 131332 A (HITACHI MEDICAL CORP), 20. Mai 1997 (1997-05-20)

## Beschreibung

Die Erfindung liegt auf dem Gebiet der Magnetresonanz-Technologie, insbesondere auf dem Gebiet bildgebender Kernspintomographen.

Die Erfindung bezieht sich auf eine Hochfrequenz-Empfangsspulenanordnung für die Untersuchung eines Untersuchungsobjekts, insbesondere eines Patienten, in einem Kernspintomographie-Gerät.

Die Erfindung betrifft außerdem ein Kernspintomographie-Gerät zur Untersuchung eines Patienten, mit einem Mittel zur Erzeugung eines statischen Magnetfelds, welches vertikal bezüglich der Patientenachse orientiert ist.

Bei einem Kernspintomographie-Gerät für medizinische Anwendungen, insbesondere für die medizinische Diagnose, wird der zu untersuchende Patient in der Regel horizontal flach liegend in den Untersuchungsbereich eingeführt. Das in dem Untersuchungsbereich erforderliche starke statische Magnetfeld wird entweder mittels eines Permanentmagneten oder mittels einer elektrischen, vorzugsweise supraleitenden, Spule erzeugt. Bei einem Kernspintomographie-Gerät mit einem Permanentmagneten, welcher zur Erzeugung kleiner oder mittlerer Feldstärken verwendet wird, ist das Magnetfeld in Folge der medizinisch bedingten horizontalen Lagerung des Patienten und der daraus resultierenden Aufstellung des Magneten geodätisch vertikal orientiert. Man spricht von einem Vertikalfeld-Gerät. Demgegenüber ist bei Verwendung einer elektrischen Magnetspule, mittels der besonders hohe Feldstärken erreichbar sind, das statische Magnetfeld parallel zur Patientenachse, also horizontal, orientiert.

Zum Senden von Hochfrequenzimpulsen in das Untersuchungsobjekt und zum Empfangen von aus dem Untersuchungsobjekt stammenden elektromagnetischen Hochfrequenzsignalen wird eine Sende- oder Empfangsspule verwendet. Die meisten der verwendeten Spulen lassen sich hinsichtlich ihrer Feldverteilung in zwei Kategorien einteilen: Volumenspulen einerseits, die ein homogenes magnetisches Feld in einem Bereich innerhalb der Spule erzeugen, und Oberflächenspulen andererseits, die ein mehr oder weniger inhomogenes Magnetfeld außerhalb der Spule erzeugen. Bei einer Volumenspule ist daher das Untersuchungsobjekt in der Regel im Spuleninneren angeordnet, wogegen eine Oberflächenspule zumeist an der Oberfläche des Untersuchungsobjekts angebracht oder aufgelegt wird. Eine typische Volumenspule mit im Inneren weitestgehend homogener Feldverteilung ist beispielsweise ein Solenoid, also eine auf eine Zylinderfläche gewickelte Drahtspule koaxial zur Patientenachse. Das Solenoid könnte auch als Schleifenspule mit einer Vielzahl von Leiterschleifen bezeichnet werden. Eine Schleifenspule mit nur einer oder sehr wenigen Leiterschleifen könnte sowohl als Oberflächenspule verwendet werden, andererseits aber auch noch als Volumenspule mit reduzierter Feldhomogenität bezeichnet werden, insbesondere, falls die Leiterschleifen das Untersuchungsobjekt einschließen.

Zum Erzielen einer möglichst homogenen Feldverteilung über das Messvolumen wird zum Senden der Hochfrequenzimpulse in der Regel eine Volumenspule oder eine entsprechende andere Antenne mit homogener Feldverteilung verwendet.

Volumenspulen haben insbesondere dann, falls nur ein bestimmter Teilbereich des Untersuchungsobjekts, insbesondere des menschlichen Körpers, abgebildet werden soll, den Nachteil, dass wegen des geringen Füllfaktors das Signal-Rausch-Verhältnis der Kernresonanzuntersuchung negativ beeinflusst ist. Als Empfangsspulen werden daher, insbesondere dann, wenn sich der zu untersuchende Teilbereich nicht von einer Spule umschließen läßt, Oberflächenspulen verwendet, das heißt, man opfert die Homogenität der Hochfrequenz-Empfangscharakteristik teilweise zugunsten eines größeren Füllfaktors, um ein lokal möglichst gutes Signal-Rausch-Verhältnis zu erhalten.

In einem Vertikalfeld-Gerät ist das bildgebende, aus dem Untersuchungsobjekt stammende elektromagnetische Hochfrequenzsignal mit seiner zirkularen Polarisation (Magnetfeldkomponente) im Wesentlichen horizontal orientiert. Eine Methode zum Empfang des zirkular polarisierten Hochfrequenzsignals besteht darin, dass zwei orthogonale Komponenten, hiervon z. B. eine erste Polarisationskomponente parallel zur Patientenachse und eine zweite Polarisationskomponente senkrecht zur Patientenachse, gesondert empfangen werden.

Zum Empfang der parallel zur Patientenachse orientierten ersten Polarisationskomponente kann eine Schleifen- oder Loop-Spule verwendet werden, die den gesamten Patientenkörper oder eine zu untersuchende Gliedmaße umschließt. Dabei ist der Durchmesser des zu untersuchenden Objekts durch den von der Schleifenspule eingeschlossenen Innenbereich nach oben beschränkt. Es wurden daher bisher Schleifenspulen mit verschiedenen Durchmessern verwendet, wobei für den jeweiligen Patienten eine passende Spule ausgesucht wurde. Diese Arbeit ist für das medizinische Personal unangenehm, da jeweils elektrische Kontaktverbindungen erneut hergestellt werden müssen. Außerdem kann es bei einem extrem adipösen Patienten vorkommen, dass keine der vorhandenen Spulen über ausreichenden Durchmesser verfügt. Dieses Problem tritt insbesondere bei einer Wirbelsäulendarstellung auf, da hierbei die Schleifenspule den gesamten Patientenkörper einschließen muss und nicht nur eine Extremität (Arm oder Bein).

Aus der JP H09 131 332 A ist eine Ausgestaltung einer Hochfrequenz-Empfangsspulenanordnung bekannt, bei welcher auf einer Trägerstruktur Spulenteile einer Oberflächenspule angeordnet sind. Die Spulenteile sind in sich offen und nicht miteinander verbunden. Die Spulenteile weisen Kontakte auf. Auf die Kontakte können alternativ Überbrückungsteile oder Kontaktelemente aufgesetzt werden. Je nachdem, ob auf die Kontakte die Überbrückungsteile oder die Kontaktelemente aufgesetzt werden, wird entweder eine Oberflächenspule oder eine Schleifenspule gebildet. Aus der JP H09 131 332 A ist weiterhin eine alternative Gestaltung bekannt, bei welcher sowohl die Kontaktelemente als auch die Überbrückungsteile vorhanden sind. In diesem Fall sind Schalteinrichtungen vorhanden, mittels derer von einer Steuereinrichtung alternativ die Kontaktelemente oder die Überbrückungsteile aktiv geschaltet werden.

Aus der US 6 169 400 B ist eine Schleifenspule bekannt, die an einer Stelle öffenbar ist.

Aus der DE 39 32 648 A1 ist eine Hochfrequenz-Empfangsspulenanordnung bekannt, bei welcher auf ein Unterteil ein Oberteil aufsetzbar ist. Wenn das Oberteil nicht auf das Unterteil aufgesetzt ist, ist ausschließlich ein im Unterteil angeordneter erster Empfangskreis aktiv. Wenn das Oberteil auf das Unterteil aufgesetzt ist, ist zusätzlich zum ersten Empfangskreis ein im Oberteil angeordneter zweiter Empfangskreis aktiv.

Der Erfindung liegt die Aufgabe zugrunde, eine Hochfrequenz-Empfangsspulenanordnung anzugeben, bei der die Zahl der notwendigen Empfangsspulenwechsel vermindert ist, und die insbesondere auch zur Untersuchung extrem adipöser Patienten geeignet ist.

Die Aufgabe wird durch eine Hochfrequenz-Empfangsspulenanordnung gemäß Anspruch 1 gelöst.

Als besonderer Vorteil ergibt sich, dass die Schleifenspule derart auf der Trägerstruktur angeordnet ist, dass das Umschließen des Untersuchungsobjekts von der Schleifenspule durch Biegen der Trägerstruktur erreichbar ist. Insbesondere ist die Hochfrequenz-Empfangsspulenanordnung im unverbogenen oder planen Zustand der Trägerstruktur zum Auflegen auf oder Unterschieben unter den Patienten geeignet. Im verbogenen Zustand ist die Schleifenspule gebildet. Dadurch ergibt sich der Vorteil, dass mit ein und derselben, von dem Bedienpersonal handzuhabenden Trägerstruktur sowohl eine Oberflächenspule als auch eine das Volumen des Patienten einschließende Spule realisiert ist.

Bezüglich der Begriffe "Oberflächenspule" und "Schleifenspule" wird auf die eingangs gemachten Erläuterungen verwiesen. Die Oberflächenspule ist insbesondere flach ausgeführt und zum Auflegen oder seitlichen Anlegen an das Untersuchungsobjekt oder den Patienten geeignet. Die Schleifenspule ist insbesondere zum Einschließen des Untersuchungsobjekts geeignet. In diesem Sinne kann die Schleifenspule auch als Volumenspule bezeichnet werden. Die Schleifen- oder Windungszahl, und somit die Ausdehnung der Schleifenspule entlang ihrer zentralen Spulenachse, ist vorzugsweise erheblich geringer als die Breitenausdehnung. Mit anderen Worten: Die Schleifenspule ist ebenfalls flach ausgebildet. Sie weist beispielsweise eine einzige oder - insbesondere bei einer kleinen Spule - 2, 3, 4 oder bis zu 10 Leiterschleifen auf.

Dadurch, dass bei der Hochfrequenz-Empfangsspulenanordnung nach der Erfindung sowohl eine Oberflächenspule als auch eine Schleifenspule zum Empfang ein und derselben Polarisationskomponente vorhanden sind, ergibt sich der Vorteil, dass auch bei großvolumigen Untersuchungsobjekten immer wenigstens eine verwendbare Empfangsspule vorhanden ist. Dies ist die Oberflächenspule.

Die Oberflächenspule hat zwar den Nachteil, dass sie im Vergleich zur Schleifenspule, insbesondere bei einem schlanken Patienten, ein niedrigeres Signal-Rausch-Verhältnis erzielt. Dieser Nachteil geht allerdings mit dem besonderen Vorteil einher, dass durch das Vorhandensein der Oberflächenspule die Hochfrequenz-Empfangsspulenanordnung nach der Erfindung auch dann ein Hochfrequenzsignal für Kernspintomographie-Untersuchungen liefert, falls die Schleifenspule nicht mehr verwendbar ist. Dies ist z.B. bei einem besonders adipösen Patienten oder bei einem Patienten mit Verletzungen oder aufgebrachtem Verbandmaterial nicht mehr möglich. Die Hochfrequenz-Empfangsspulenanordnung nach der Erfindung ist somit in ihrer Anwendung nicht durch die Größe oder die Beschaffenheit des Patientenumfangs begrenzt. Andererseits kann bei einem schlanken Patienten die Schleifenspule mit (unverändert) hoher Bildqualität eingesetzt werden.

Die erste Polarisationskomponente kann also wahlweise entweder mit der Oberflächenspule oder mit der Schleifenspule gemessen werden.

Die Oberflächenspule ist vorzugsweise als Butterfly-Spule oder als Sattelspule ausgebildet. Bezüglich der Details dieser Spulenvarianten wird auf das Buch von Marinus Ir. Vlaardingerbroek, Jacques A. den Boer, "Magnetic Resonance Imaging", Springer Berlin, 1996, Seite 32 bis 38, verwiesen.

Vorzugsweise ist ein Abschnitt der zumindest einen Leiterbahn sowohl Teil der Oberflächenspule als auch Teil der Schleifenspule.

Nach einer bevorzugten Weiterbildung der Hochfrequenz-Empfangsspulenanordnung ist eine weitere, ebenfalls auf der Trägerstruktur angeordnete Oberflächenspule vorhanden, die zum Empfang einer zweiten Polarisationskomponente des Hochfrequenzsignals ausgebildet ist, wobei die zweite Polarisationskomponente senkrecht zur ersten Polarisationskomponente orientiert ist. Die weitere Oberflächenspule ist bevorzugt als Butterfly-Spule oder als Sattelspule ausgebildet. Bei Vorhandensein der weiteren Oberflächenspule ergibt sich der Vorteil, dass beide Feldkomponenten des zirkular polarisierten Magnetfeldanteils des elektromagnetischen Hochfrequenzsignals detektierbar sind.

Vorzugsweise ist die Trägerstruktur für eine lokale Untersuchung des Patienten tragbar ausgeführt.

Die erfindungsgemäße Hochfrequenz-Empfangsspulenanordnung kann insbesondere bei einem Kernspintomographie-Gerät verwendet werden, das als Vertikalfeld-Gerät ausgebildet ist. In diesem Fall ist die erste Polarisationskomponente parallel zur Patientenachse orientiert.

Vier Ausführungsbeispiele einer Hochfrequenz-Empfangsspulenanordnung und eines Kernspintomographie-Geräts nach der Erfindung werden nachfolgend anhand der Figuren 1 bis 7 näher erläutert. Es zeigen:
- Figur 1: ein Kernspintomographie-Gerät nach der Erfindung in einer schematischen Gesamtansicht,
- Figur 2: eine Hochfrequenz-Empfangsspulenanordnung für das Kernspintomographie-Gerät gemäß einer ersten Ausführungsform,
- Figur 3: eine Hochfrequenz-Empfangsspulenanordnung für das Kernspintomographie-Gerät gemäß einer zweiten Ausführungsform,
- Figur 4: eine Hochfrequenz-Empfangsspulenanordnung für das Kernspintomographie-Gerät gemäß einer dritten Ausführungsform,
- Figur 5: ein Ausführungsbeispiel für die elektrische Beschaltung der Empfangskanäle der Hochfrequenz-Empfangsspulenanordnung,
- Figur 6: eine mögliche, nicht erfindungsgemäße elektrische Beschaltung der Empfangskanäle der Hochfrequenz-Empfangsspulenanordnung und
- Figur 7: eine Hochfrequenz-Empfangsspulenanordnung für das Kernspintomographie-Gerät gemäß einer vierten Ausführungsform.

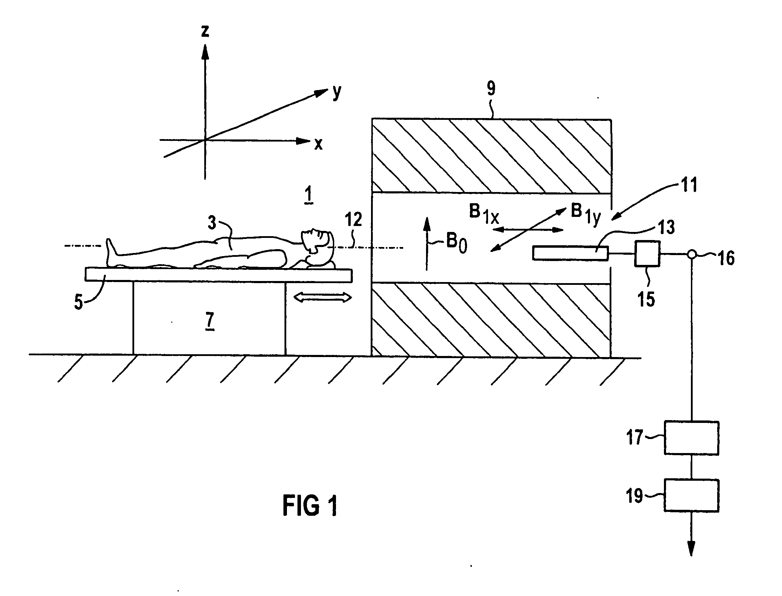

**Figur 1** zeigt ein insgesamt mit dem Bezugszeichen 1 bezeichnetes Kernspintomographie-Gerät, mittels welchem ein Bereich eines Untersuchungsobjekts oder eines Patienten 3 bildgebend untersucht werden soll. Der Patient ist in einem Zustand vor Beginn der Untersuchung dargestellt, in welchem er auf einer von einem Support 7 getragenen, horizontal verschiebbaren Liege 5 aufgelegt ist.

Das Kernspintomographie-Gerät 1 weist zur Erzeugung eines statischen Magnetfelds B₀ einen Permanentmagneten 9 auf, in dessen Öffnung 11 die eigentliche Untersuchung stattfindet. Mittels einer nicht explizit dargestellten Sendespule werden Hochfrequenzimpulse in den in die Öffnung 11 eingeführten Patienten 3 eingestrahlt. Aus dem Patienten 3 stammende Echoimpulse werden von einer Hochfrequenz-Spulenanordnung 13 aufgenommen und über einen Vorverstärker 15 und einen Verstärker 17 einem Analog-Digital-Wandler 19 zugeführt. In Figur 1 ist eine Anschlussstelle 16 als Bezugspunkt für die nachfolgenden Figuren eingezeichnet.

Das von der Hochfrequenz-Spulenanordnung 13 empfangene Hochfrequenzfeld ist mit seinem Magnetfeldanteil in der Horizontalebene (x-y) zirkular polarisiert. Die entsprechenden Feldkomponenten werden als erste Polarisationskomponente B₁ₓ bzw. als zweite Polarisationskomponente B_{1y} bezeichnet.

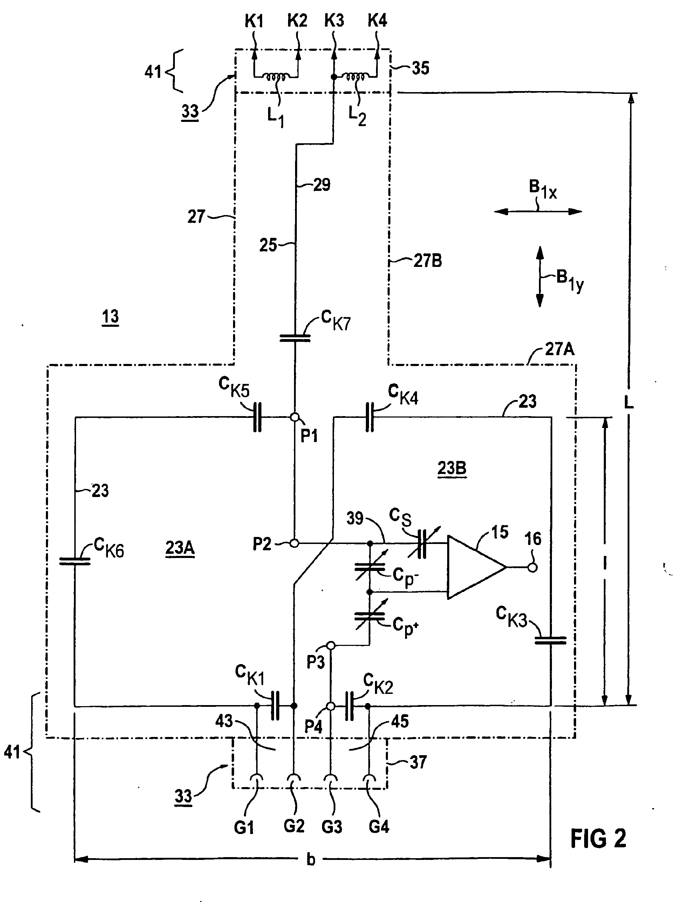

In **Figur 2** ist die Hochfrequenz-Spulenanordnung 13 der Figur 1 gemäß einer ersten Ausführungsform dargestellt. Die Hochfrequenz-Spulenanordnung 13 umfasst eine als Butterfly-Spule ausgebildete Oberflächenspule 23 und eine eine einzige Schleife oder Windung (Loop) aufweisende Schleifenspule 25. Die beiden Spulen 23, 25 sind auf einer gemeinsamen, nur angedeuteten Trägerstruktur 27 aus weichem, flexiblem Material, vorzugsweise Kunststoff, aufgebracht.

Die Trägerstruktur 27 umfasst ein flächiges Basisteil 27A sowie ein längs ausgedehntes Ringteil 27B. Die Oberflächenspule 23 ist im Basisteil 27A angebracht. Die Schleifenspule 25 erstreckt sich sowohl durch das Basisteil 27A als auch durch das Ringteil 27B, wobei durch ein Verbiegen des in Figur 2 noch plan dargestellten Ringteils 27B (aus der Zeichenebene heraus) zu einem Ring eine geschlossene Leiterschleife 29 der Schleifenspule 25 realisierbar ist.

Hierzu ist ein Verbindungsmittel 33 vorhanden, das einen an dem Ringteil 27B endseitig befestigten Stecker 35 mit elektrischen Kontakten K1, K2, K3, K4 sowie eine entsprechende, am Basisteil 27A befestigte Buchse 37 mit entsprechend passenden Gegenkontakten G1, G2, G3, G4 umfasst. Bei gesteckter oder verbundener Schleifenspule 25 ist der Kontakt K3 mit dem entsprechenden Gegenkontakt G3 verbunden, so dass eine die Punkte P1, P2, P3, P4 aufweisende, mehr oder weniger ringförmige Leiterschleife 29 gebildet ist.

Die Oberflächenspule 23 umfasst einen linksseitigen Spulenteil 23A sowie einen rechtsseitigen Spulenteil 23B, die durch sich überkreuzende Leitungen, quasi in Form einer "Acht" miteinander verbunden sind (Butterfly-Spule).

Sowohl in die Oberflächenspule 23 als auch in die Schleifenspule 25 sind sogenannte Verkürzungskondensatoren C_{K1}, C_{K2}, C_{K3}, C_{K4}, C_{K5}, C_{K6} bzw. C_{K7} integriert, die beispielsweise eine Kapazität von 200 pF aufweisen.

Der Leitungsabschnitt P1-P2-P3-P4 ist sowohl Teil der Oberflächenspule 23 als auch Teil der Schleifenspule 25. An den Punkten P2, P3 sind die Empfangssignale aus beiden Spulen 23, 25 über eine gemeinsame Leitung 39 abführbar. Zur Impedanzanpassung der Leitung 39 an die Spulen 23, 25 ist ein gemeinsamer Anpasskondensator C_{S} Vorhanden. Gemeinsame Stimmkondensatoren Cp-, C_{P}+ erlauben ein Anpassen der Spulenresonanzfrequenz an die Larmorfrequenz des Hochfrequenzfeldes.

Über die Leitung 39 sind die Empfangssignale dem Vorverstärker 15 zuführbar. In Figur 2 ist die Leitung 39 aus Gründen der Übersichtlichkeit als an dem Punkt 16 endend dargestellt, der auch in Figur 1 wiedergegeben ist.

Das bereits erwähnte Verbindungsmittel 33, umfassend den Stecker 35 und die Buchse 37, ist Bestandteil einer Umschalteinrichtung 41 zum abwechselnden Deaktivieren und/oder Aktivieren der Oberflächenspule 23 und der Schleifenspule 25. Die Funktion dieser Umschalteinrichtung 41 wird nachfolgend für das Beispiel des Aktivierens der Schleifenspule 25 und dem gleichzeitigen Deaktivieren der Oberflächenspule 23 erläutert: Durch Stecken des Steckers 35 in die Buchse 37 wird - wie bereits erläutert - über den Kontakt K3 und den Gegenkontakt G3 die Leiterschleife 29 der Schleifenspule 25 geschlossen und die Schleifenspule 25 somit bildgebend aktiviert. Zum Deaktivieren der Oberflächenspule 23 werden die Kontakte K1 bis K4 und die Gegenkontakte G1 bis G4 verwendet. Hierzu umfasst die Umschalteinrichtung 41 eine erste Sperrspule L₁ (L₁ ≈ 0,08pF) sowie eine zweite Sperrspule L₂ (z.B. L₂ ≈ 0,08µF), die in dem Stecker 35 zwischen jeweils zwei Kontakten K1, K2 bzw. K3, K4 angeschlossen sind. Durch das Einfügen des Steckers 35 in die Buchse 37 werden Schwingkreise 43, 45 geschlossen, die durch den Verkürzungskondensator C_{K1} und die erste Sperrspule L₁ bzw. durch den Verkürzungskondensator C_{K2} und die zweite Sperrspule L₂ gebildet werden. Die auf die Resonanzfrequenz des Kernspintomographen 1 abgestimmten Schwingkreise 43, 45 ((L₁· C_{K1)}^{-1/2} = (L₂· C_{K2})^{-1/2} = W_{Larmor} = 2π f_{Larmor}) wirken als Sperrkreis und unterbinden somit weitestgehend den Stromfluss in der Oberflächenspule 23. Mit dem linksseitigen Schwingkreis 43 wird das linksseitige Spulenteil 23A und mit dem rechtsseitigen Schwingkreis 45 gesondert das rechtsseitige Spulenteil 23B abgeschaltet. Die Oberflächenspule 23 ist dann verstimmt und nicht mehr bildgebend aktiv.

Zur Erzielung einer Sperrwirkung in der Oberflächenspule 23 können alternativ zu den Schwingkreisen 43, 45 mechanische Unterbrecher, beispielsweise Schalter oder Drucktaster vorhanden sein, die bei Einführen des Steckers 35 in die Buchse 37 betätigt werden.

Die Breite b der Oberflächenspule 23 beträgt etwa 250 mm und ihre Länge 1 ebenfalls ca. 250 mm. Die Länge L der Leiterschleife 29, welche den maximalen Umfang der Schleifenspule 25 festlegt, beträgt ca. 560 mm.

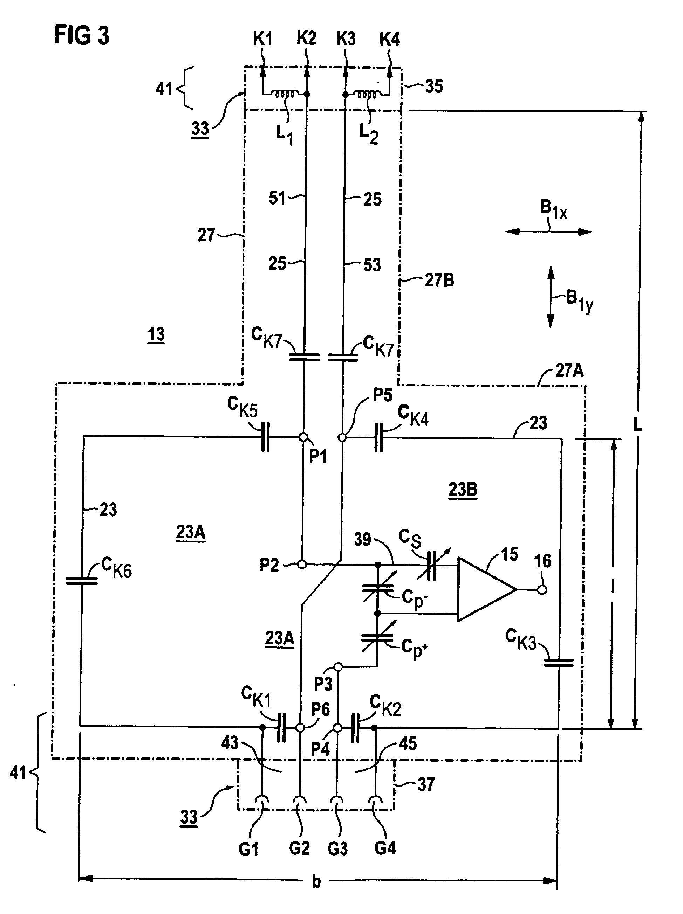

Die in **Figur 3** dargestellte zweite Ausführungsform der Hochfrequenz-Spulenanordnung 13 ist weitestgehend mit der in Figur 2 dargestellten identisch. In diesem Beispiel weist die Schleifenspule 25 allerdings zwei seriell miteinander verbundene Leiterschleifen 51, 53 auf, die mittels des Kontakts K2 und des Gegenkontakts G2 bzw. mittels des Kontakts K3 und des Gegenkontakts G3 gesondert steckbar sind.

Bei der in Figur 3 dargestellten Ausführungsform sind die Leitungsabschnitte P1-P2-P3-P4 und P5-P6 sowohl zur Oberflächenspule 23 als auch zur Schleifenspule 25 gehörig.

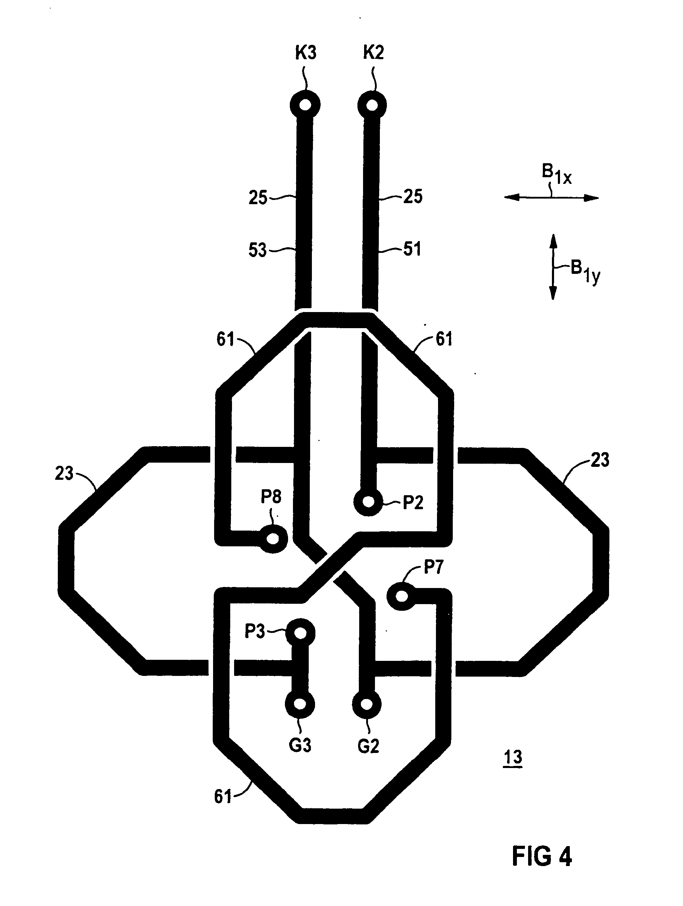

Bei der in **Figur 4** dargestellten dritten Ausführungsform einer Hochfrequenz-Spulenanordnung 13 nach der Erfindung ist zusätzlich zu der Oberflächenspule 23 und der Schleifenspule 25, die zur Detektion der ersten Polarisationskomponente B₁ₓ (parallel zur Patientenachse 12) verwendet werden, eine weitere Oberflächenspule 61 vorhanden, die ebenfalls als Butterfly-Spule ausgebildet ist. Die beiden Oberflächenspulen 23, 61 sind gekreuzt und um 90° gegeneinander verdreht angeordnet, so dass mit der weiteren Oberflächenspule 61 die oben bereits erwähnte zweite Polarisationskomponente B_{1y} detektierbar ist.

In Figur 4 ist die Hochfrequenz-Spulenanordnung 13 schematisch in einer Form dargestellt, wie sie in etwa auf einer flexiblen Platine durch chemisches Ätzen realisiert ist. An den Punkten P2, P3 werden die Empfangssignale der für die erste Polarisationskomponente B₁ₓ zuständigen Spulen 23, 25 abgenommen, an gesonderten Punkten P7, P8 die von der für die zweite Polarisationskomponente B_{1y} zuständigen Spule 61 erzeugten Signale. Bei der schematischen Darstellung der Figur 4 sind Schaltungsdetails, wie Kondensatoren, und die Umschalteinrichtung 41 aus Gründen der Übersichtlichkeit weggelassen. Auch ist die Länge L der Leiterschleife 29 nicht maßstabgerecht gezeichnet.

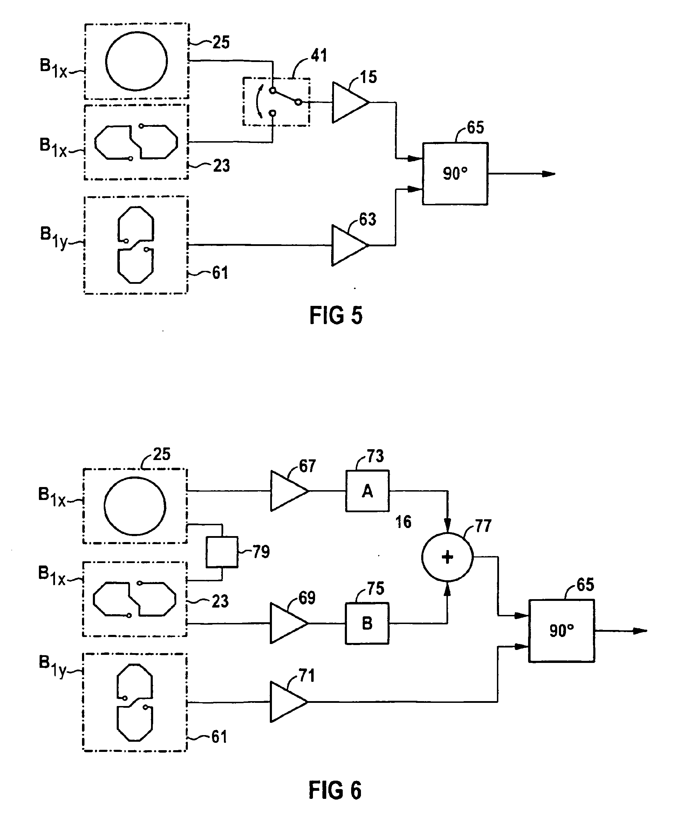

In den Figuren 5 und 6 ist beispielhaft dargestellt, wie die Empfangssignale in der in Figur 4 dargestellten Hochfrequenz-Spulenanordnung 13 mit insgesamt drei Spulen 23, 25, 61 weiterverarbeitet werden. Die beiden Figuren geben die Beschaltung der Spulen 23, 25, 61 nur funktionell wieder, ohne dass elektronische Details angegeben sind.

Wie in **Figur 5** dargestellt ist, werden die Ausgangssignale der Oberflächenspule 23 und der Schleifenspule 25 unter Verwendung der Umschalteinrichtung 41 abwechselnd dem Vorverstärker 15 zugeführt. Das Empfangssignal der weiteren Oberflächenspule 61 gelangt über einen ihr zugeordneten gesonderten Vorverstärker 63 zu einem 90°-Koppler (90°-Combiner) 65, der die 90°-Phasenverschiebung zwischen den beiden Polarisationskomponenten B₁ₓ, B_{1y} berücksichtigt.

**Figur 6** zeigt eine nicht zur Erfindung gehörige Spulenanordnung, bei der die Spulen 23, 25 zur Messung der ersten Polarisationskomponente B₁ₓ simultan betrieben werden. Ihre Ausgangssignale werden über Vorverstärker 67 bzw. 69 verstärkt. Hier unterscheidet sich die Beschaltung von der der Figuren 2 und 3. Die verstärkten Signale werden in Multiplizierern 73 bzw. 75 mit Wichtungsfaktoren A, B multipliziert und einer Summiereinrichtung 77 zugeführt. Das Ausgangssignal der Summiereinrichtung 77 gelangt zusammen mit dem Ausgangssignal eines der weiteren Oberflächenspule 61 zugeordneten gesonderten Vorverstärkers 71 zum 90°-Koppler 65.

Damit die gleichzeitig resonanten Spulen 23, 25 zur Messung der ersten Polarisationskomponente B₁ₓ sich nicht nachteilig gegenseitig beeinflussen, ist eine Anordnung 79 zur elektrischen Entkopplung der beiden Spulen 23, 25 vorhanden. Die Anordnung 79 zur elektrischen Entkopplung umfasst z.B. einen Kondensator, dessen Wert so gewählt ist, dass die gemeinsame Induktivität kompensiert ist.

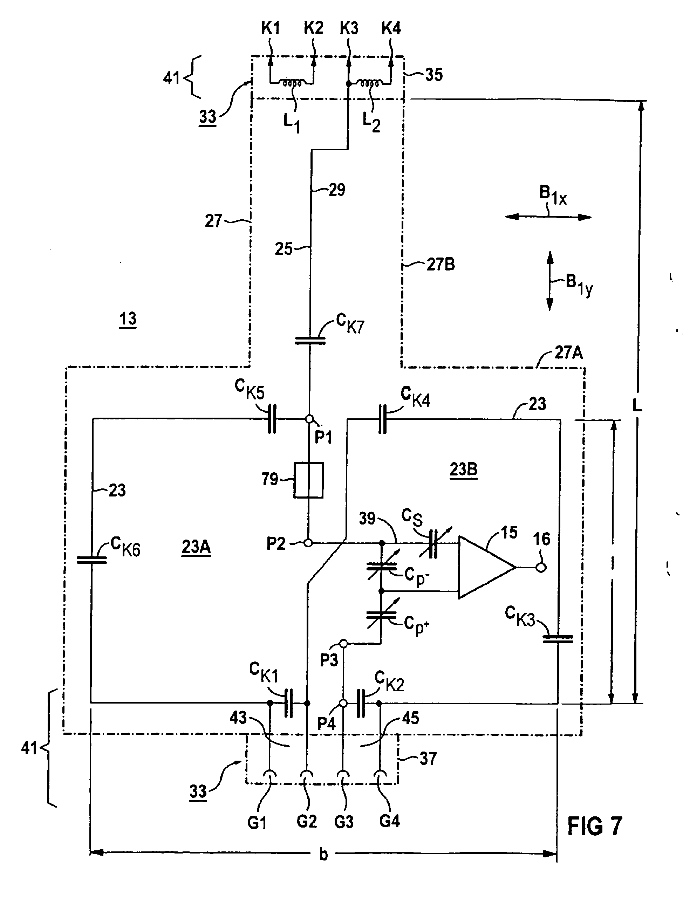

Die Anordnung 79 zur elektrischen Entkopplung kann auch dann vorhanden sein, falls die Spulen 23, 25, wie in Figur 5 dargestellt, mittels der Umschalteinrichtung 41 wahlweise bildgebend aktiviert werden. In entsprechender Abänderung des in Figur 2 dargestellten Ausführungsbeispiels ist dies in **Figur 7** gezeichnet.

## Patentansprüche

1. Hochfrequenz-Empfangsspulenanordnung für die Untersuchung eines Untersuchungsobjekts (3) in einem Kernspintomographie-Gerät (1),
- welche eine biegsame, flächige Trägerstruktur (27) aufweist, auf der Leiterbahnen zur Ausbildung von sowohl einer Schleifenspule (25) und einer Oberflächenspule (23) als auch von deren Verbindungen (39) zu einem gemeinsamen Vorverstärker (15) angeordnet sind,
- wobei an einem ersten Ende der Trägerstruktur (27) ein Stecker (35) mit mehreren elektrischen Kontakten (K1, K2, K3, K4) und an einem zweiten Ende der Trägerstruktur (27), welches dem ersten Ende diametral gegenüberliegt, eine Buchse (37) mit entsprechenden elektrischen Gegenkontakten (G1, G2, G3, G4) angeordnet sind;
- wobei zumindest eine der Leiterbahnen (29) sich auf der Trägerstruktur (27) zwischen Stecker (35) und Buchse (37) erstreckt;
- wobei aus einem planen Zustand der flächigen Trägerstruktur (27) heraus zumindest ein Teil (27B) der flächigen Trägerstruktur (27) um das Untersuchungsobjekt (3) herum derart biegbar ist, dass im gebogenen Zustand dieses Teils (27B) der Stecker (35) und die Buchse (37) eine lösbare Verbindung bilden;
- wobei im planen Zustand der Trägerstruktur (27) die Oberflächenspule (23) bildgebend aktiviert ist;
- wobei demgegenüber im gebogenen Zustand der Trägerstruktur (27) durch eine geeignete Verbindung von Kontakten (K1, K2, K3, K4) und Gegenkontakten (G1, G2, G3, G4) von Stecker (35) und Buchse (37) die zumindest eine Leiterbahn, die sich auf der Trägerstruktur zwischen Stecker (35) und Buchse (37) erstreckt, ringförmig geschlossen ist, wodurch sie die Schleifenspule (25) bildet, welche dabei aufgrund der Verbindung von Stecker (35) und Buchse (37) bildgebend aktiviert ist, während gleichzeitig die Oberflächenspule (23) bildgebend deaktiviert ist, indem der Stromfluss in der Oberflächenspule (23) durch am Stecker (35) und an der Buchse (37) vorgesehene Sperrmittel, entweder in Form von sich durch Verbindung des Steckers (35) mit der Buchse (37) schließenden sperrenden Schwingkreisen (43, 45) oder in Form von durch dieses Einführen betätigten mechanischen Unterbrechern, unterbrochen ist;
- und wobei die Schleifenspule (25) und die Oberflächenspule (23) zum Empfang einer gleichen ersten Polarisationskomponente (B₁ₓ) eines aus dem Untersuchungsobjekt (3) stammenden elektromagnetischen Hochfrequenzsignals ausgebildet sind.

2. Hochfrequenz-Empfangsspulenanordnung nach Anspruch 1, bei der die Oberflächenspule (23) als Butterfly-Spule oder als Sattelspule ausgebildet ist.

3. Hochfrequenz-Empfangsspulenanordnung nach Anspruch 1 oder 2, bei der ein Abschnitt (P1-P2-P3-P4) der zumindest einen Leiterbahn (29) sowohl Teil der Oberflächenspule (23) als auch Teil der Schleifenspule (25) ist.

4. Hochfrequenz-Empfangsspulenanordnung nach Anspruch 1 oder 2, bei der eine weitere, ebenfalls auf der Trägerstruktur (27) angeordnete Oberflächenspule (61) vorhanden ist, die zum Empfang einer zweiten Polarisationskomponente (B_{1y}) des Hochfrequenzsignals ausgebildet ist, wobei die zweite Polarisationskomponente (B_{1y}) senkrecht zur ersten Polarisationskomponente (B₁ₓ) orientiert ist.

5. Hochfrequenz-Empfangsspulenanordnung nach Anspruch 4, bei der die weitere Oberflächenspule (61) als Butterfly-Spule oder als Sattelspule ausgebildet ist.

6. Hochfrequenz-Empfangsspulenanordnung nach einem der Ansprüche 1 bis 5, bei der für eine lokale Untersuchung eines Patienten (3) die Trägerstruktur (27) tragbar ausgeführt ist.

7. Kernspintomographie-Gerät zur Untersuchung eines Patienten (3), mit einem Mittel zur Erzeugung eines statischen Magnetfelds (B₀), welches vertikal bezüglich der Längsachse (12) des Patienten (3) orientiert ist, und mit einer Hochfrequenz-Empfangsspulenanordnung (13) nach einem der Ansprüche 1 bis 6, bei der die erste Polarisationskomponente (B₁ₓ) parallel zur Längsachse (12) des Patienten (3) orientiert ist.

## Claims

1. Radio-frequency reception coil arrangement for examining an object under examination (3) in a nuclear magnetic resonance imaging device (1),
- which arrangement has a flexible, flat carrier structure (27) on which conductor tracks for forming both a loop coil (25) and a surface coil (23) and their connections (39) to a common preamplifier (15) are arranged,
- a connector (35) with a plurality of electrical contacts (K1, K2, K3, K4) being arranged at a first end of the carrier structure (27) and a socket (37) with corresponding electrical mating contacts (Gl, G2, G3, G4) being arranged at a second end of the carrier structure (27) which is diametrically opposite the first end;
- at least one of the conductor tracks (29) extending on the carrier structure (27) between the connector (35) and the socket (37);
- at least one part (27B) of the flat carrier structure (27) being able to be bent around the object under examination (3) from a planar state of the flat carrier structure (27) in such a manner that the connector (35) and the socket (37) form a releasable connection in the bent state of this part (27B);
- the surface coil (23) being activated in terms of imaging in the planar state of the carrier structure (27);
- the at least one conductor track which extends on the carrier structure between the connector (35) and the socket (37) in contrast being annularly closed in the bent state of the carrier structure (27) by means of a suitable connection between contacts (K1, K2, K3, K4) and mating contacts (Gl, G2, G3, G4) of the connector (35) and the socket (37), as a result of which the conductor track forms the loop coil (25) which is activated in terms of imaging in this case on account of the connection between the connector (35) and the socket (37), while the surface coil (23) is simultaneously deactivated in terms of imaging by virtue of the flow of current in the surface coil (23) being interrupted by blocking means provided on the connector (35) and on the socket (37), either in the form of blocking resonant circuits (43, 45) which are closed by the connection of the connector (35) to the socket (37) or in the form of mechanical interrupters which are actuated by this insertion;
- and the loop coil (25) and the surface coil (23) being designed to receive an identical first polarization component (B₁ₓ) of an electromagnetic radio-frequency signal coming from the object under examination (3).

2. Radio-frequency reception coil arrangement according to Claim 1, in which the surface coil (23) is in the form of a butterfly coil or saddle coil.

3. Radio-frequency reception coil arrangement according to Claim 1 or 2, in which a section (P1-P2-P3-P4) of the at least one conductor track (29) is both part of the surface coil (23) and part of the loop coil (25).

4. Radio-frequency reception coil arrangement according to Claim 1 or 2, in which there is a further surface coil (61) which is likewise arranged on the carrier structure (27) and is designed to receive a second polarization component (B_{1y}) of the radio-frequency signal, the second polarization component (B_{1y}) being oriented perpendicular to the first polarization component (B₁ₓ).

5. Radio-frequency reception coil arrangement according to Claim 4, in which the further surface coil (61) is in the form of a butterfly coil or saddle coil.

6. Radio-frequency reception coil arrangement according to one of Claims 1 to 5, in which the carrier structure (27) is designed to be portable for local examination of a patient (3).

7. Nuclear magnetic resonance imaging device for examining a patient (3), having a means for producing a static magnetic field (B₀), which is oriented vertically with respect to the longitudinal axis (12) of the patient (3), and having a radio-frequency reception coil arrangement (13) according to one of Claims 1 to 6, in which the first polarization component (B₁ₓ) is oriented parallel to the longitudinal axis (12) of the patient (3).

## Revendications

1. Ensemble de bobines de réception haute fréquence pour l'examen d'un objet à examiner (3) dans un appareil pour tomographie à spin nucléaire,
- ledit ensemble présentant une structure de support (27) plane et souple sur laquelle sont agencées des pistes conductrices pour constituer non seulement une bobine en boucle (25) et une bobine de surface (23), mais aussi leurs liaisons (39) avec un préamplificateur commun (15) ;
- un connecteur (35) présentant plusieurs contacts électriques (K1, K2, K3, K4) étant situé à une première extrémité de la structure de support (27) et une prise femelle (37) présentant des contacts électriques opposés correspondants (Gl, G2, G3, G4) étant située à une deuxième extrémité de la structure de support (27) qui est diamétralement opposée à la première extrémité ;
- au moins l'une des pistes conductrices (29) s'étendant sur la structure de support (27) entre le connecteur (35) et la prise femelle (37) ;
- au moins une partie (27B) de la structure de support plane (27) pouvant, à partir d'un état plat de la structure de support plane (27), être pliée de manière telle autour de l'objet à examiner (3) que, à l'état plié de cette partie (27B), le connecteur (35) et la prise femelle (37) forment un assemblage amovible ;
- la bobine de surface (23) étant, à l'état plat de la structure de support (27), activée en termes d'imagerie ;
- l'au moins une piste conductrice qui s'étend sur la structure de support entre le connecteur (35) et la prise femelle (37) étant, en revanche, fermée annulairement à l'état plié de la structure de support (27) du fait d'un assemblage approprié de contacts (K1, K2, K3, K4) et de contacts opposés (Gl, G2, G3, G4) du connecteur (35) et de la prise femelle (37), de sorte qu'elle forme la bobine en boucle (25), celle-ci étant activée en termes d'imagerie en raison de l'assemblage du connecteur (35) et de la prise femelle (37), tandis que la bobine de surface (23) est en même temps désactivée en termes d'imagerie du fait de l'interruption du flux de courant dans la bobine de surface (23) par des moyens de blocage prévus sur le connecteur (35) et sur la prise femelle (37), soit sous la forme de circuits résonants de blocage (43, 45) qui se ferment du fait de l'assemblage du connecteur (35) et de la prise femelle (37) ou sous la forme d'interrupteurs mécaniques actionnés par cette introduction ; et
- la bobine en boucle (25) et la bobine de surface (23) étant conçues pour recevoir une même première composante de polarisation (B₁ₓ) d'un signal électromagnétique haute fréquence provenant de l'objet à examiner (3).

2. Ensemble de bobines de réception haute fréquence selon la revendication 1, dans lequel la bobine de surface (23) se présente sous la forme d'une bobine papillon ou d'une bobine en forme de selle.

3. Ensemble de bobines de réception haute fréquence selon la revendication 1 ou 2, dans lequel un tronçon (P1-P2-P3-P4) de l'au moins une piste conductrice (29) fait partie non seulement de la bobine de surface (23), mais aussi de la bobine en boucle (25).

4. Ensemble de bobines de réception haute fréquence selon la revendication 1 ou 2, dans lequel on a une autre bobine de surface (61) qui est également située sur la structure de support (27) et qui est conçue pour recevoir une deuxième composante de polarisation (B_{1y}) du signal haute fréquence, la deuxième composante de polarisation (B_{1y}) étant orientée verticalement par rapport à la première composante de polarisation (B₁ₓ).

5. Ensemble de bobines de réception haute fréquence selon la revendication 4, dans lequel l'autre bobine de surface (61) se présente sous la forme d'une bobine papillon ou d'une bobine en forme de selle.

6. Ensemble de bobines de réception haute fréquence selon l'une des revendications 1 à 5, dans lequel la structure de support (27) est réalisée de manière portative pour un examen local d'un patient (3).

7. Appareil pour tomographie à spin nucléaire destiné à l'examen d'un patient (3), comportant des moyens pour générer un champ magnétique statique (B₀) qui est orienté verticalement par rapport à l'axe longitudinal (12) du patient (3), et un ensemble de bobines de réception haute fréquence (13) selon l'une des revendications 1 à 6, dans lequel la première composante de polarisation (B₁ₓ) est orientée parallèlement à l'axe longitudinal (12) du patient (3).
